# EUROPEAN PATENT SPECIFICATION

(11) **EP 3 946 585 B1**
(45) Date of publication and mention of the grant of the patent: **19.10.2022**
(21) Application number: 20713658.1
(22) Date of filing: 27.03.2020
(51) Int. Cl.: A61N 5/10

(54) **DETERMINING HEMATOLOGIC TOXICITY RISK FOLLOWING RADIOTHERAPY**
BESTIMMUNG DES RISIKOS EINER HÄMATOLOGISCHEN TOXIZITÄT NACH EINER STRAHLENTHERAPIE
DÉTERMINATION DU RISQUE DE TOXICITÉ HÉMATOLOGIQUE APRÈS UNE RADIOTHÉRAPIE

(30) Priority: 28.03.2019 EP 19165769
(43) Date of publication of application: 09.02.2022
(73) Proprietor: Koninklijke Philips N.V., 5656 AG Eindhoven (NL)
(72) Inventor: DA SILVA RODRIGUES, Pedro, Jorge, 5656 AE Eindhoven (NL); BONDAR, Maria, Luiza, 5656 AE Eindhoven (NL); VITTORINO DE ALMEIDA, Vanda, Lucia, De, Carvalho, 5656 AE Eindhoven (NL)
(74) Representative: Philips Intellectual Property & Standards
(86) International application number: PCT/EP2020/058843
(87) International publication number: WO 2020/193788

(56) References cited:
- WO-A1-01/10461
- WO-A2-2015/116932
- US-B2- 9 925 393

## Description

### FIELD OF THE INVENTION

Embodiments described herein generally relate to methods, systems and computer program products for determining a risk of hematologic toxicity. The methods are particularly applicable to determining such risk in response to radiotherapy treatment. For instance, the method may be applied to determine at each radiotherapy fraction a risk that a subject will develop an acute hematological toxicity. As a consequence, the method can also be used to determine which patients will be at risk for hematological toxicity at the end of the radiotherapy course.

### BACKGROUND OF THE INVENTION

Rapid and severe lymphopenia (a reduction of blood circulating lymphocytes) occurs within 30 days in 50% of patients that undergo radiotherapy treatments, in either standalone radiotherapy (RT), sequential (sCRT) or concurrent to systemic chemotherapy (cCRT) ("Standard radiotherapy but not chemotherapy impairs systemic immunity in non-small cell lung cancer, Yazdi et al, Oncoimmunology, 2016, VOL. 5, NO. 12, e1255393, "Review of hematological indices of cancer patients receiving combined chemotherapy & radiotherapy or receiving radiotherapy alone", Shahid et al, Critical Reviews in Oncology/Hematology 105 (2016) 145-155, "Treatment-related Lymphopenia in Patients With Stage III Non-Small-Cell Lung Cancer", Campian et al, Cancer Invest. 2013 March). This may typically occur in subjects treated with RT for solid tumours such as, but not limited to, e.g, locally advanced non-small cell lung cancer, locally advanced head and neck and glioblastoma. Traditional fractionated radiation can hinder immune responses in part due to death of lymphocytes in the radiation field leading to systemic lympho-depletion. In a standard 1.8 - 2 Gy, 28 - 30 fractions RT regime, 99% of all circulating lymphocytes receive more than the LD50 of 0.5 Gy (mean lymphocyte absorbed dose is 2.2 Gy).

Besides lymphopenia (also described in the medical literature as lymphocytopaenia), other acute haematological toxicities (HT) can develop, such as neutropenia, leukopenia (including lymphopenia), anaemia and thrombocytopenia. Clinical evidence reports that these acute haematological toxicities develop either within the classical six to eight weeks of radiotherapy treatment window or during the follow-up period. Recent research indicate that this HT results from irradiation of bone marrow or/and blood vessels present within the treatment fields. Neutropenia (depletion of neutrophils) can usually be resolved within 28-30 days, although prolonged and high-grade lymphopenia (higher than grade two Common Terminology Criteria for Adverse Events (CTCAE), duration from 1 month and up to 2-3 years) in 50% of all patients has been reported.

It is today understood that for an effective tumor control and coupling with other therapies like checkpoint inhibitor immunotherapies, the CD8+ T cell population needs to be preserved. While baseline CD8 lymphopenia that resolves within the first months of therapy has not been directly correlated with anti-PDl/anti-PD-Ll therapy outcomes (in contrast to the neutrophils-to-lymphocytes ratio), prolonged CD4+/CD8+ lymphopenia (like that triggered by classic radiotherapy, or concurrent chemo-radiotherapy), defined as abnormal low numbers of CD4+ and CD8+ lymphocytes for more than 3 months, has been linked to sub-optimal outcomes in anti-PD-1/anti-PD-L1 therapies ("Association between pretreatment lymphocyte count and response to PD1 inhibitors in head and neck squamous cell carcinomas", Ho et al, Journal for ImmunoTherapy of Cancer (2018) 6:84, "Relationships between lymphocyte counts and treatment-related toxicities and clinical responses in patients with solid tumors treated with PD-1 checkpoint inhibitors" Diehl et al, Oncotarget, 2017, Vol 8, (No 69), pp:114268-114280).

An indication of the contribution of radiation-induced lymphopenia to the outcomes of combined CRT and anti-PD-L1 therapies can be found in the published results of the PACIFIC trial ("Integrating immunotherapy into chemoradiation regimens for medically inoperable locally advanced non-small cell lung cancer", Jabbour et al, Transl. Lung Cancer Res. 2017 Apr;6(2):113-118). This Phase 3 clinical trial established the combination of CRT with an anti-PD-Ll antibody (durvalumab, administered for 1 year after the end of CRT) for the treatment of LA-NSCLC (standard of care since September 2017 in the USA). Originally, in the PACIFIC trial, patients should start anti-PD-Ll within 14 days (recommended value that arises from the typical dynamics of the adaptive immune system) after last RT fraction. However, during the trial, the period was amended to 42 days (Protocol for: Antonia SJ, Villegas A, Daniel D, et al. Durvalumab after chemoradiotherapy in stage III non-small cell lung cancer. N Engl J Med 2017;377:1919-29) although patients that were treated within 14 days had better benefits.

The increase of the time to 42 days was due to the need to resolve any toxicities from CRT before the start of the anti-PD-Ll agent ("Immunotherapy for Unresectable Stage III Non-Small-Cell Lung Cancer", Rizvi and Peters, N Engl J Med 377;20 November 16, 2017). Based on the recovery time for the different immune cell compartments recovery, it is expected that the 42 days should have provided enough time to improve the total number of neutrophils but in some of the patients a persistent reduction of lymphocyte should still be present at day 42.

Awareness of the importance of the immune system in the response to radiotherapy, chemo-radiotherapy and triple chemo-radiotherapy-immunotherapy combination regimes has recently led to attempts to reduce the hematologic toxicities associated with such treatments. Recent results indicate that thoracic vertebral (TV) irradiation contributes to acute hematologic toxicity during CRT in LA-NSCLC. This hematologic toxicity represents one of main causes for treatment interruption and therefore sub-optimal outcomes, due to the inability to provide the required RT fractions within the prescribed time which has a direct relation to tumour control. It is now advocated that sparing irradiation to thoracic vertebra (and consequently bone marrow) should be considered, with limits to the mean vertebra dose of around 24 Gy, volume at 20 Gy less than 56% and volume at 30 Gy less than 52% ("Thoracic Vertebral Body Irradiation Contributes to Acute Hematologic Toxicity During Chemoradiation Therapy for Non-Small Cell Lung Cancer" Deek et al, Int J Radiat Oncol Biol Phys. 2016 January 01; 94(1): 147-154).

Document WO 2015/116932 discusses a method for determining a risk of haematologic toxicity in a subject to be treated with radiotherapy, the method comprising:
- receiving patient data associated with the subject;
- receiving treatment data,wherein the treatment data includes a prescribed dose of radiation to be administered to the subject
- receiving imaging data, wherein the imaging data comprises an image including blood;
- processing the treatment data and the imaging data to determine a received dose of radiation to be delivered to the radiation-sensitive tissues in the subject
- processing the patient data and the received dose of radiation to obtain a risk of haematologic toxicity in the subject in response to the radiotherapy.

However, even if these constraints are placed within the OAR (Organs at Risk) definition during radiotherapy planning, there is no accounting/tracking of the actual dose received by the thoracic bones (and consequently the bone marrow), other lymphoid organs such as the thymus and large vessels during each fraction. Currently, patient positioning in the radiotherapy linear accelerator is made via external markers complemented by anatomic images (usually Cone Beam CT) on-board imagers. Due to possibly independent motion of the articulated bony anatomy components with respect to each other, there is no guarantee that the thoracic bones are in the same position of the original CT that is used as input for radiotherapy planning. Note that although bone misalignment can be corrected at patient setup, corrections are limited to rigid correction only (and often corrections are limited to translational corrections only). In addition, it is possible that for several treatment fractions, the positioning of the patient in respect to the beam would be driven by the primary tumor location (in order to maximize tumour control) sacrificing a possible extra dose to the bone marrow and other lymphoid organs ("Emerging Therapies for Stage III Non-Small Cell Lung Cancer: Stereotactic Body Radiation Therapy and Immunotherapy" Front Oncol. 2017 Sep 4;7:197.).Thus in current clinical practice, no account is taken of the actual absorbed dose or the probability to develop an HT during or after the CRT period.

Accordingly there is a need for additional methods for determining a risk of hematologic toxicity in subjects to be treated with radiotherapy. This issue is particularly important for therapies in which radiotherapy is combined with a sequential immunotherapy treatment, as in the PACIFIC regime.

### SUMMARY OF THE INVENTION

The object of the present invention is solved by the subject matter of the independent claims where further embodiments are incorporated in the dependent claims. It should be noted that the following described aspects of the invention apply equally to the method for determining a risk of hematologic toxicity, to the system and to the computer program product.

In this context and according to one aspect, there is provided a computer-implemented method for determining a risk of hematologic toxicity in a subject to be treated with radiotherapy. The method comprises a step of receiving patient data associated with the subject, wherein the patient data includes blood cell counts in the subject. The method further comprises a step of receiving treatment data, wherein the treatment data includes a prescribed dose of radiation to be administered to the subject. The method further comprises a step of receiving imaging data, wherein the imaging data comprises an image including one or more radiation-sensitive tissues in the subject, the radiation-sensitive tissues comprising regions of hematopoeisis and/or leukocyte maturation, activation, and/or proliferation in the subject. The radiation-sensitive tissues comprise bone marrow and/or lymphoid organs. The method further comprises a step of processing the treatment data and the imaging data to determine a received dose of radiation to be delivered to the radiation-sensitive tissues in the subject. The method further comprises a step of processing the patient data and the received dose of radiation to obtain a risk of hematologic toxicity in the subject in response to the radiotherapy.

In one embodiment, the hematologic toxicity comprises lymphopenia and the patient data includes lymphocyte numbers in the subject's blood.

In one embodiment, the patient data further comprises age, gender and/or disease characteristics in the subject.

In one embodiment, the treatment data further comprises a distribution of radiation doses to be administered to treated areas within the subject.

In one embodiment, the imaging data is derived from computerized tomography (CT), positron emission tomography (PET), single photon computerized tomography (SPECT) or magnetic resonance imaging (MRI). Preferably the imaging data is indicative of proliferative and/or metabolic activity in the radiation-sensitive tissues of the subject. For instance, the imaging data may be derived from PET imaging using suitable tracers such as 18F-fluorothymidine (18F-FLT), 18F-fluorodeoxyglucose (18F-FDG), or 89Zr-Df-IAB22M2C.

In one embodiment, the radiation-sensitive tissue comprises bone marrow. Preferably, the radiation-sensitive tissue comprises bone marrow of the thoracic bones.

In one embodiment, the radiotherapy comprises fractionated radiotherapy. Preferably the treatment data includes a plurality of fraction doses. Preferably the imaging data comprises a plurality of images, wherein each image is obtained at administration of a different radiotherapy fraction.

In one embodiment, the received dose of radiation to be delivered to radiation-sensitive tissues in the subject is outputted to a display.

In one embodiment, the method further comprises receiving updated patient data including blood cell counts from the subject obtaining after initiation of the radiotherapy treatment. The updated patient data may be received, for example, if the risk of hematologic toxicity is determined to be above a predetermined threshold. In this embodiment, the method may further comprise obtaining an updated risk of hematologic toxicity in the subject based on the updated patient data, treatment data and imaging data.

In one embodiment, the subject is to be treated with combined radiotherapy and immunotherapy for cancer.

In a further aspect, the present invention provides a system for determining a risk of hematologic toxicity in a subject to be treated with radiotherapy. The system comprises an interface, a memory and a processor.

The interface is configured for receiving (i) patient data associated with the subject, wherein the patient data includes blood cell counts in the subject; (ii) treatment data, wherein the treatment data includes a prescribed dose of radiation to be administered to the subject; and
(iii) imaging data, wherein the imaging data comprises a graphical representation including one or more radiation-sensitive tissues in the subject, the radiation-sensitive tissues comprising regions of hematopoeisis and/or leukocyte maturation, activation, and/or proliferation in the subject; wherein the radiation-sensitive tissues comprise bone marrow and/or lymphoid organs;
The processor is configured to execute instructions stored on the memory to (a) process the treatment data and the imaging data to determine a received dose of radiation to be delivered to the radiation-sensitive tissues in the subject; and
(b) process the patient data and the received dose of radiation to obtain a risk of hematologic toxicity in the subject in response to the radiotherapy.

In a further aspect, the present invention provides a computer program product comprising a non-transitory computer readable medium, the computer readable medium having computer readable code embodied therein, the computer readable code being configured such that, on execution by a suitable computer or processor, the computer or processor is caused to perform the method as described above.

### BRIEF DESCRIPTION OF THE DRAWINGS

Exemplary embodiments of the invention will now be described with reference to the following drawings wherein:
Fig. 1 shows a systematic block diagram of a computerized system for determining a risk of hematologic toxicity; and
Fig. 2 shows a flow chart of a method for determining a risk of hematologic toxicity in a subject to be treated with radiotherapy.
Fig. 3 illustrates one embodiment of an overview of the present method.
Fig. 4 shows an embodiment of the present method in which a risk of hematologic toxicity is calculated after a number of fractions of radiotherapy treatment.

### DETAILED DESCRIPTION OF EMBODIMENTS

In embodiments of the present invention, a method is provided to compute (e.g. at each radiotherapy fraction) the risk that a patient will develop an acute hematologic toxicity (e.g. for a specific grade), including for example neutropenia, leukopenia, lymphopenia, anaemia or thrombocytopenia. The method may compute such a risk based on irradiation of radiation-sensitive tissues (such as bone marrow, other lymphoid organs such as the thymus, heart or blood vessels) present within the treatment fields with a defined radiation dose. A graphical user interface (GUI) may be provided to inform the user of the risk that the patient will develop an acute hematologic toxicity. The provided GUI may allow the user to visualise the anatomical location of radiation-sensitive tissues (such as bone marrow) inside the patient and assess the extent of damage at these locations.

Dose tracking to the radiation-sensitive tissues (e.g. bone marrow such as in thoracic vertebrae) within the radiation treatment field may be useful in predicting the degree of destruction of e.g. bone marrow stem cells, and therefore a likelihood of development of HT. In addition, in embodiments of the present invention assessments of blood immune cell populations are made, e.g. at baseline, in the middle of the treatment and at the end thereof. The combination of measurements of circulating immune cell populations with a surrogate that represents the capability of bone marrow to generate new cells can thereby improve the estimation of HT risk during therapy. The method may be used e.g. by a clinician to improve awareness of the effect of TV patient positioning at each fraction, or to predict and plan the appropriate supportive care in case of high risk of HT development. This is especially valuable e.g. in combination CRT-immunotherapies for LA-NSCLC, where each 1-year of treatment of durvalumab has a list price of 180 000 dollars per patient (total number of eligible patients worldwide is 500 000 per year).

Thus in particular embodiments, the invention as described herein may comprise one or more of the following general features:
(a) a method that estimates the absorbed radiation dose to bone marrow, for each fraction i, of a total number of fractions N of a radiotherapy treatment protocol, as well as the total received dose up to fraction i. The dosimetric quantities are used as surrogate for the destruction of bone marrow stem cells, which will affect the production of new white cells such as neutrophils and lymphocytes in a certain time after irradiation.
(b) a graphical user interface (GUI) that displays the relevant dosimetric quantities (for example dose-volume-histograms, DVH) or a color wash of the accumulated dose volume overlaid to the different bone marrow locations.
(c) a HT risk model that as a function of the accumulated dose to different bone marrow locations provides an estimate of the risk of appearance of toxicities within a time window (for example, 15 days from the current fraction i, 15 days after the last radiotherapy fraction). The model may be characterized by a certain number of input parameters and estimates the probability of HT. The input parameters may include, for example, the dose targets for bone marrow (for example thoracic vertebra), other lymphoid organs (for example the thymus), heart, heart chambers, lung, major blood vessels, radiotherapy delivery parameters (e.g. dose rate, beam angles, beam-on-time), geometrical and image features of irradiated regions, and the blood panel parameters measured at baseline before the start of the radiotherapy treatment.

If the risk of HT is determined to be above user-defined values, the user may be instructed to acquire a new set of blood panel from the patient. The updated blood panel values (for example, numbers of neutrophils or lymphocytes) are used to update the HT risk model and reflect the fast dynamics expected in the circulating white cells due to the absorbed radiation (e.g. lethal dose 50 for CD8+ lymphocytes is 0.5 Gy).

Various embodiments are described more fully below with reference to the accompanying drawings, which form a part hereof, and which show specific exemplary embodiments. However, the concepts of the present disclosure may be implemented in many different forms and should not be construed as limited to the embodiments set forth herein; rather, these embodiments are provided as part of a thorough and complete disclosure, to fully convey the scope of the concepts, techniques and implementations of the present disclosure to those skilled in the art. Embodiments may be practiced as methods, systems or devices. Accordingly, embodiments may take the form of a hardware implementation, an entirely software implementation or an implementation combining software and hardware aspects. The following detailed description is, therefore, not to be taken in a limiting sense.

Reference in the specification to "one embodiment" or to "an embodiment" means that a particular feature, structure, or characteristic described in connection with the embodiments is included in at least one example implementation or technique in accordance with the present disclosure. The appearances of the phrase "in one embodiment" in various places in the specification are not necessarily all referring to the same embodiment.

Unless specifically stated otherwise as apparent from the following discussion, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system memories or registers or other such information storage, transmission or display devices. Portions of the present disclosure include processes and instructions that may be embodied in software, firmware or hardware, and when embodied in software, may be downloaded to reside on and be operated from different platforms used by a variety of operating systems. The invention may take form in various components and arrangements of components, and in various process operations and arrangements of process operations.

The present disclosure also relates to an apparatus for performing the operations herein. This apparatus may be specially constructed for the required purposes, or it may comprise a general-purpose computer selectively activated or reconfigured by a computer program stored in the computer. Such a computer program may be stored in a computer readable storage medium, such as, but is not limited to, any type of disk including floppy disks, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each may be coupled to a computer system bus. Furthermore, the computers referred to in the specification may include a single processor or may be architectures employing multiple processor designs for increased computing capability.

The processes and displays presented herein are not inherently related to any particular computer or other apparatus. Various general-purpose systems may also be used with programs in accordance with the teachings herein, or it may prove convenient to construct more specialized apparatus to perform one or more method steps. The structure for a variety of these systems is discussed in the description below. In addition, any particular programming language that is sufficient for achieving the techniques and implementations of the present disclosure may be used. A variety of programming languages may be used to implement the present disclosure as discussed herein.

In addition, the language used in the specification has been principally selected for readability and instructional purposes and may not have been selected to delineate or circumscribe the disclosed subject matter. Accordingly, the present disclosure is intended to be illustrative, and not limiting, of the scope of the concepts discussed herein. Numerous additional advantages and benefits will become apparent to those of ordinary skill in the art upon reading the following detailed description.

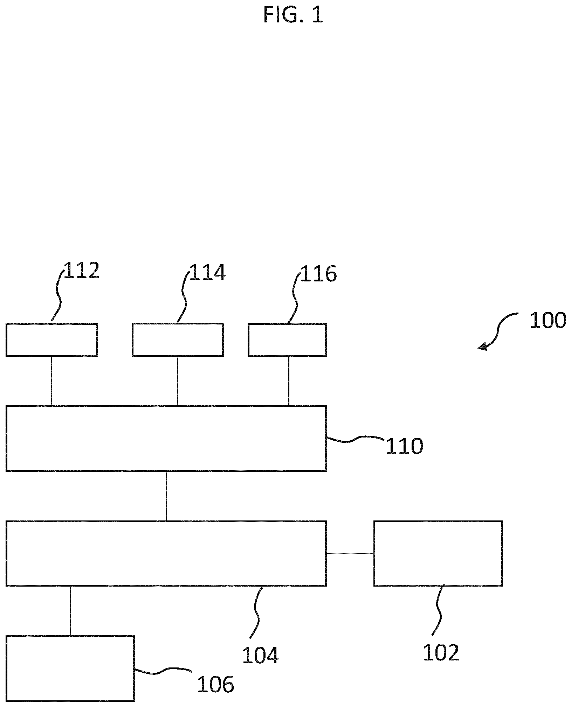

Fig. 1 illustrates a system 100 for determining a risk of hematologic toxicity in a subject to be treated with radiotherapy in accordance with one embodiment.

The system 100 may include a user input/output (I/O) device 102 and a processor 104 executing instructions stored on memory 106. The processor 104 may be in communication with or otherwise include an interface 110 for receiving data from data sources 112, 114 and 116. The data may be associated with a subject to treated with radiotherapy, e.g. the data may relate to clinical characteristics and/or diagnostic test results associated with the subject.

Data source 112 may comprise a database comprising patient clinical data. In particular, the data comprises blood cell counts relating to the subject, e.g. a number of leukocytes or subtypes thereof (e.g. lymphocytes) per unit volume of blood in the subject. The data may further comprise patient characteristics such as age, gender, race, comorbidities and so on. The data may further comprise disease characteristics relating to the subject, e.g. the nature of the cancer from which the subject is suffering, including features such as tumor size, stage and histological parameters.

Data source 114 may comprise treatment data relating to a radiotherapy treatment to be applied to the subject. For instance, the treatment data may include details of the proposed treatment including a dose of radiation prescribed by a physician, a treatment plan including fractions of the dose to be delivered at particular times/dates, and a distribution of radiation doses to be administered to particular areas within the subject.

Data source 116 may comprise a source of imaging data relating to the subject, typically relating to *in vivo* functional imaging of the subject. For instance, data source 116 may comprise a functional or anatomic imaging system such a positron emission tomography (PET) scanner, a magnetic resonance imaging (MRI) scanner, computerized tomography (CT), and/or single-photon emission computed tomography (SPECT).

The I/O device 102 may be any suitable device that can receive commands from an operator and output processed data graphically and/or in any other form. The I/O device 102 may be configured as, for example but without limitation, a personal computer, a tablet, laptop, mobile device, or the like.

The processor 104 may be any specifically configured processor or hardware device capable of executing instructions stored on memory 106 to process biological data in order to determine quantitative values therefrom. The processor 104 may include a microprocessor, a field programmable gate array (FPGA), application-specific integrated circuit (ASIC), or other similar device. In some embodiments, such as those relying on one or more ASICs, the functionality described as being provided in part via software may instead be hardwired into the operation of the ASICs, and as such, any associated software may be omitted.

The memory 106 may be LI, L2, L3 cache or RAM memory configurations. The memory 106 may include non-volatile memory such as flash memory, EPROM, EEPROM, ROM, and PROM, or volatile memory such as static or dynamic RAM, as discussed above. The exact configuration/type of memory 106 may of course vary as long as instructions for analyzing biological data can be executed by the processor 104.

The interface 110 may receive biological data from the data sources 112, 114 and/or 116. The interface 110 may then communicate the received data to the processor 104 for analysis. The biological data may be, for example, in the form of patient and treatment data relating to the subject, as well as digital images obtained by functional and anatomic (e.g. CT, SPECT, PET or MRI) imaging of the subject.

The processor 104 is configured to calculate a received dose of radiation that will be delivered to radiation-sensitive tissues in the subject, based on the received treatment and imaging data. For example, the processor may analyse the imaging data to identify regions of hematopoiesis and/or leukocyte proliferation such as bone marrow within the image. The processor then determines, based on the treatment data, how much of the prescribed dose of radiation will be delivered to such radiation-sensitive tissues. The processor 104 is further configured to determine a risk of hematologic toxicity in the subject, based on the dose of radiation delivered to the radiation-sensitive tissues and the patient data, in particular the baseline blood cell counts in the subject.

After analysis of the received data, the processor 104 may output the obtained risk of hematologic toxicity in the subject to the I/O device 102 or another display unit. In some embodiments, the processor may calculate a risk of each grade of hematologic toxicity (e.g. lymphopenia grades 1 to 5), and output the calculated values to the I/O device. The output may be in the form of a list of risks, a ranking table, a treatment recommendation, or e.g. a graphical representation of any of the above.

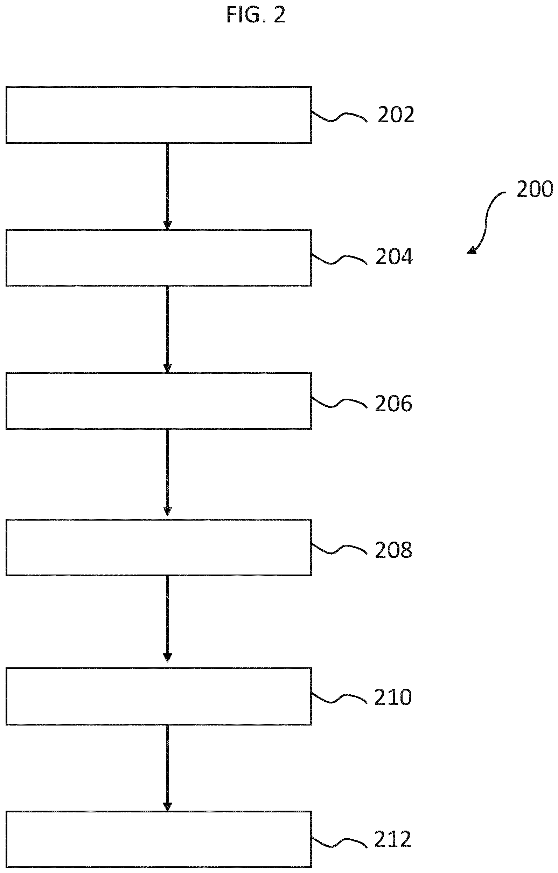

Fig. 2 depicts a flowchart of a method 200 for determining a risk of hematologic toxicity in a subject to be treated with radiotherapy using the system of FIG. 1 in accordance with one embodiment. Step 202 involves receiving patient data (e.g. blood cell counts) associated with the subject. Step 204 comprises receiving treatment data such as a prescribed dose of radiation to be administered to the subject. Step 206 involves receiving imaging data relating to the subject, in particular images that show radiation-sensitive tissues (such as bone marrow) in the subject.

The patient data, treatment data and imaging data may be received by the interface 110 from the data sources 112, 114 and/or 116. A processor such as the processor 104 of FIG. 1 may receive these images from the interface 110 of FIG. 1. In alternative embodiments, the patient data, treatment data and/or imaging data may be transmitted to the interface 110 and/or processor 104 by the I/O device 102, e.g. where the data is stored in another location after data acquisition by the data sources.

Step 208 involves processing the treatment data and the imaging data to determine a received dose of radiation to be delivered to the radiation-sensitive tissues in the subject. For instance, the processor 104 may use an algorithm suitable for analysis of digital images to identify radiation-sensitive tissues in the image. In one embodiment, the imaging data is derived from PET imaging using a tracer indicative of cell proliferation, e.g. 18F-fluorothymidine or indicative of metabolic activity, e.g. 18F-fluorodeoxyglucose. In this way, the processor can identify radiation-sensitive tissues (e.g. in bone marrow showing active hematopoiesis) present in the image. The processor then uses the treatment data to determine whether the identified radiation-sensitive tissues are within radiotherapy treatment field, and to thereby calculate the dose of radiation delivered to such tissues.

Step 210 involves processing the received dose of radiation obtained in step 208 and the patient data to obtain a risk of hematologic toxicity in the subject in response to the radiotherapy. For instance, the processor may calculate the extent to which normal hematopoiesis will be disrupted by the dose of radiation received by the radiation-sensitive tissues, and thus predict how circulating leukocyte numbers will change over time in response to the radiotherapy. This calculation may be made based on known data indicating the relationship between irradiation of hematopoietic tissues at particular doses and levels of hematopoiesis (e.g. of lymphocytes and/or neutrophils), or may be determined using machine learning techniques. For instance, the processor may access a database of clinical data showing treatment data, imaging data and patient data (e.g. blood cell counts before and after radiotherapy treatment) of a plurality of individual subjects, and thus may learn the relationship between received doses of radiation in radiation-sensitive tissues and subsequent leukopenia (e.g. lymphopenia and/or neutropenia). By using the patient data including baseline blood cell counts, the processor may thus calculate a risk that a hematologic toxicity (including e.g. lymphopenia) will develop in the subject.

In one embodiment, in order to train a toxicity prediction model, the grade and the type of toxicity is collected from a population of patients treated with RT at a specific point in time during or after the completion of the treatment course. For example grade 3 and larger than 3 (grade 3+) lymphopenia after 1 week, or 4 weeks after RT, grade 2+ neutropenia at 2 weeks, etc. A model is trained and validated on retrospective patient data and used to predict toxicity for a new patient.

There are two standard machine learning approaches to modelling risk of side effects for RT applications. The first approach uses analytical phenomenological models of the impact of irradiation on organs at risk (for example the Lyman-Kutcher-Burman (LKB) model, Kutcher G J and Burman C 1989 Calculation of complication probability factors for non-uniform normal tissue irradiation: the effective volume method Int. J. Radiat. Oncol. Biol. Phys. 16 1623-30). The second approach performs e.g. a multivariate logistic regression to model the relationship between the predictive variables and the side effect data. Compared to the first approach, the second approach allows including non-dosimetric factors as predictive variables in a predictive model. (I. El Naqa et al., "Dose response explorer: an integrated open-source tool for exploring and modelling radiotherapy dose-volume outcome relationships," Phys. Med. Biol., vol. 51, no. 22, pp. 5719-5735, Nov. 2006). Other machine learning models can be used to learn a relationship between side effect data and predictive variables such as neural networks, deep learning, Bayesian networks, etc.The non-uniform three-dimensional dose distribution to the organ at risk (OAR) is typically included in a risk prediction model by using a single one-dimensional (1D) dose variable D or a combination of several 1D dose variables. For example, the dose variable D can be modelled by the equivalent uniform dose (EUD), the generalized equivalent uniform dose (gEUD), the mean dose to the OAR, the median dose, the volume receiving higher than a dose value, by the weighs of the dose volume histogram in a lower dimensional space (e.g., found with principal component analysis), (I. El Naqa et al., "Dose response explorer: an integrated open-source tool for exploring and modelling radiotherapy dose-volume outcome relationships," Phys. Med. Biol., vol. 51, no. 22, pp. 5719-5735, Nov. 2006), by higher order statistics (Rossi L, Bijman R, Schillemans W, Aluwini S, Cavedon C, Witte M, Incrocci L,Heijmen B. Texture analysis of 3D dose distributions for predictive modelling of toxicity rates in radiotherapy. Radiother Oncol. 2018 Dec;129(3):548-553).

In an alternative embodiment to those using standard machine learning approaches, the processor can model the temporal dynamics of the different immune cells differentiation starting from the hematopoietic bone marrow and in-vitro radiosensitivity data for the different cell lines: stem cells, neutrophils, lymphocytes and corresponding sub-populations (CD8⁺, CD4+). Other input parameters include hematopoietic bone marrow recovery after radiation therapy. For calculation of the dose received by the circulating lymphocytes, the radiotherapy treatment planning information, that describes how and where the radiation interacts with the body and correspondent intensity, is used.

Step 212 involves providing an output via the I/O device 102 or a display to a user, e.g. a clinician. The output of the method may comprise, for example, an indication of the risk (e.g. a percentage probability) that a particular grades of e.g. lymphopenia will occur. In some embodiments, the output may include a treatment and/or diagnostic recommendation to the clinician, e.g. a recommendation that further blood cell counts are obtained and/or that the treatment protocol is modified due to a high risk of lymphopenia. In some embodiments, the output may comprise a recommended start time for immunotherapy treatment after the radiotherapy.

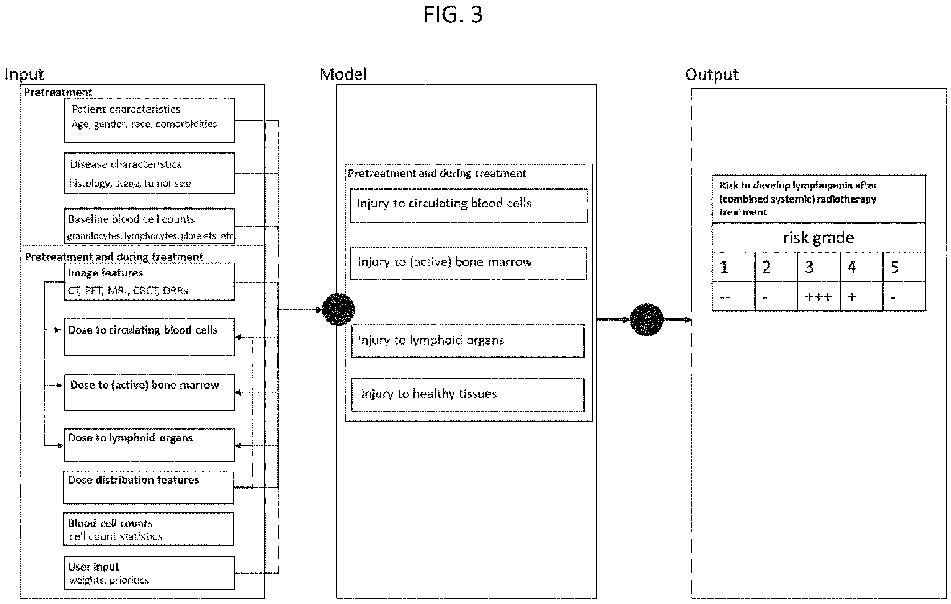

Fig. 3 illustrates one embodiment of an overview of the present method. The method comprises a step of receiving as input patient data including e.g. patient characteristics, disease characteristics and baseline blood cell counts. The method further comprises a step of receiving imaging data and treatment data, which is used in the model to determine a received dose of radiation to radiation-sensitive tissues such as bone marrow, lymphoid organs and circulating blood cells. This received dose is then used in combination with the patient data to determine injury to hematopoietic tissues and circulating blood cells, allowing the prediction of hematologic toxicity (e.g. lymphopenia). The risks of each grade of lymphopenia after exposure to the radiotherapy are then outputted, e.g. via a graphical user interface.

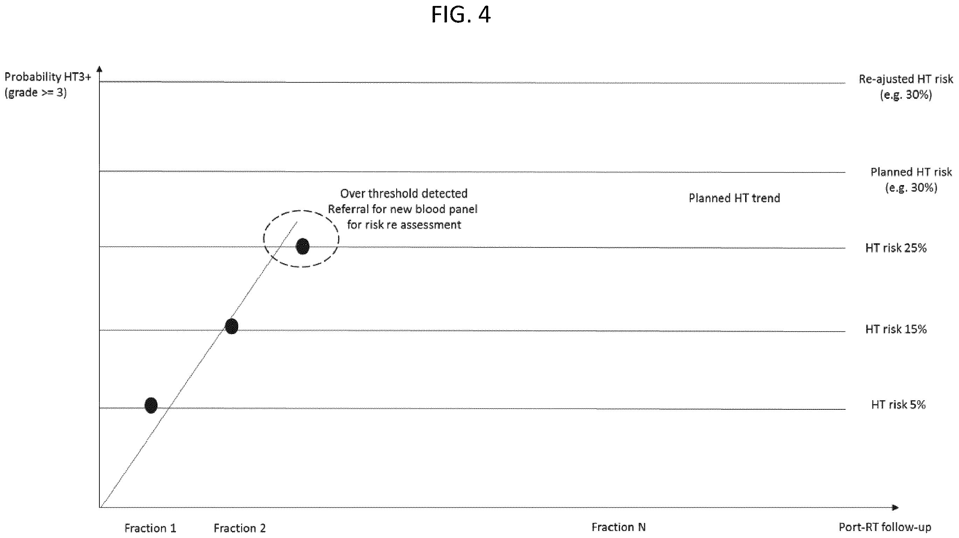

Fig. 4 shows an embodiment in which a risk of hematologic toxicity was calculated at each of 3 fractions of radiotherapy treatment protocol involving N fractions. After fraction 3 the updated risk for HT (of grade 3 or above) is already 25% in comparison with a target HT in the plan of 30%. A new blood panel is taken at fraction 3 and a new HT risk computed. In this case, an adaptation of the initial RT plan could be made with additional sparing to hematopoietic sites and additional sparing of circulating lymphocytes (reflected in the blood panel), and consequently the HT risk was downgraded.

In some embodiments, the hematologic trend (i.e. probability of hematologic toxicity after administration of increasing radiotherapy fractions, as shown in e.g. Fig. 4) may be outputted by the processor to a user interface, e.g. on a display. The hematologic risk as shown in Fig. 4 may be outputted (e.g. to a display) together with a risk estimation chart as shown e.g. in Fig. 3. In the case of the patient shown in Fig. 3 and Fig. 4 (patient 1), the most probable risk adverse grade for lymphopenia is 3. This information may then be used to schedule the patient to receive an immunotherapy agent, e.g. a checkpoint inhibitor. An example of such a scheduling protocol for 4 patients is shown in the Table below, and may be outputted on the display:

| **Patient Scheduling List for immunotherapy consolidation** | | |
|---|---|---|
| Patient ID | Risk Grade | Time to start immunotherapy after radiotherapy (time unit) |
| 76 | 1 | 5 |
| 10 | 2 | 5 |
| 1 | 3 | 60 |
| 128 | 4 | 180 |

In the case of patient 1, the start of immunotherapy is delayed by 60 days due to a relatively high risk of lymphopenia (i.e. grade 3 or above). Thus immunotherapy is only started after a significant time has passed, in order to allow for lymphopenia to resolve to normal values. In another example (patient 128) with a severe lymphopenia case (grade 4), the oncologist may decide to start the immunotherapy combination arm only later, e.g. at 180 days as recommended above.

In one example, treatment planning of NSCLC (Non-Small Cell Lung Cancer) is made taking into consideration the thoracic vertebra as an Organ-at-Risk (OAR). The optimal radiotherapy treatment plan is computed and upon approval, it will be delivered over N fractions. A baseline blood sample that provides the absolute number of neutrophils, absolute number of lymphocytes and sub-populations is collected from the patient. In one embodiment, normal tissue complication probability (NTCP) prediction relies on the Lyman-Kutcher Burman (LKB) model that includes the above mentioned input parameters ("Radiation Dose to the Thoracic Vertebral Bodies Is Associated With Acute Hematologic Toxicities in Patients Receiving Concurrent Chemoradiation for Lung Cancer: Results of a Single-Center Retrospective Analysis", Barney et al, Int J Radiation Oncol Biol Phys, Vol. 100, No. 3, pp. 748e755, 2018, "Lyman-Kutcher-Burman NTCP model parameters for radiation pneumonitis and xerostomia based on combined analysis of published clinical data", Semenenko and Li, Phys Med Biol. 2008 Feb 7;53(3):737-55, "Clinical validation of the LKB model and parameter sets for predicting radiation-induced pneumonitis from breast cancer radiotherapy", Tsougos et al, Phys. Med. Biol. 2006, 51 L1). Based on this set of parameters, an initial risk HT model is created that provides the probability of HT as a function of the absorbed dose.

At each fraction i, of a total number of fractions N, a Cone Beam CT (CBCT) image of the patient while being prepared in the linear accelerator couch for radiation treatment is acquired. Based on the CBCT images, rigid structures/bones of relevance are segmented. Using the delivered radiotherapy beam configuration, previously approved, the fraction dose, i.e., the dose absorbed by the bone marrow inside bones located within the treatment field is computed at each fraction. Then, the fraction dose is co-registered in a reference patient geometry (e.g., planning CT, or alternatively a volumetric image acquired up to fraction i). The accumulated dose up to fraction i is composed by summing up the co-registered fraction doses up to and including fraction i.

The accumulated dose-volume parameters (e.g, DVH, mean dose) for the thoracic vertebrae (TV), sternum, scapulae, clavicles, and ribs are updated. In addition, the percentage of the total organ volume exceeding × radiation dose (Vx) and mean vertebral dose (MVD) are computed as a function of the fraction. The risk computed at fraction i that an HT will develop within a certain time window, together with the relevant dosimetric input parameters (accumulated dose, Vx, MVD) is displayed.

In case the risk level is above a user-defined threshold (e.g. 20%, 30% and so on) the clinician is instructed to collect from the patient a new peripheral blood sample. The new blood panel information provides a real-time assessment of a white cell depletion due to radiation. The blood sample analysis includes panels for total counts of e.g. neutrophils and lymphocytes.

The rate of depletion of relevant white blood cells is used to weight the initial HT risk for the accumulated dose and a new HT computed. Based on the historical trend of HT, the clinician may then decide to initiate supportive care.

The patient data includes blood cell counts derived from the subject. The blood cell counts may include, for example, a number of leukocytes per unit volume in blood of the subject. In further embodiments, the blood cell counts include numbers of lymphocytes (including e.g. subsets thereof such as CD4+ and/or CD8+ T cells), granulocytes (e.g. basophils, neutrophils and/or eosinophils), monocytes and/or platelets.

Thus the hematologic toxicity, the risk of which is to be determined, may comprise leukopenia. Alternatively, the hematologic toxicity may be e.g. lymphopenia or neutropenia. In some embodiments, the method may indicate an overall risk of leukopenia in addition to a risk of e.g. lymphopenia and neutropenia.

In some embodiments, the risk of hematological toxicity may be calculated in terms of a risk of depletion of leukocyte subpopulations of interest relevant for tumor control by the immune system. For instance, the method may calculate a risk of depletion of e.g. T cell subpopulations such as CD4+ effector, CD4+ central memory, CD8+ effector and/or CD8+ central memory cells. In certain embodiments, the blood cell counts may include numbers of such cell populations in the patient's blood.

For instance in one embodiment, the method includes a CD8+ T cell functional analysis. Thus the method may involve determining a risk that CD8+ T cells are depleted. In one such embodiment, the number of functional CD8+ T cells is computed from estimates of the blood flow (for example from hemodynamic models) that cross the radiation field (described by the number of monitor units for each beam position). Blood cell counts may be used to update the number of functional CD8+ T cells after each treatment fraction, e.g. by identifying CD8+ T cells with an abnormally high number of RAD50 foci, which is indicative that these cells were inside the radiation field and are therefore expected to become apoptotic.

In another embodiment, recovery coefficients may be used in calculating the risk of hematologic toxicity. For instance, a recovery coefficient for each blood cell populations may be used to account for interruptions or completion of the radiotherapy fractionation regime, and to indicate a time taken for the blood cell populations to return to normal levels following treatment.

The imaging data may, in one embodiment, be a digital image derived from a functional *in vivo* imaging method. Thus the imaging data may be e.g. a Cone Beam CT image, a CT images, mega voltage CT image, a pseudo-CT image, a PET image, a SPECT image or an MRI image. Images acquired during or before the start of radiotherapy fraction scheme can also be used.

The radiation-sensitive tissues include regions of hematopoiesis and/or leukocyte maturation, activation and/or proliferation. The radiation-sensitive tissues include bone marrow, preferably active bone marrow, more preferably bone marrow of the thoracic vertebrae. The radiation-sensitive tissues include lymphoid organs, e.g. lymph nodes, spleen and/or thymus.

In some embodiments, the radiation-sensitive tissues include blood, i.e. a dose of radiation delivered to circulating blood cells is calculated. Thus in another embodiment, besides the bone marrow Vx and MVD dosimetric quantities, other dosimetric parameters that include regions of significant blood circulation are computed. For example lung V5, lung V10 and similar that correlate with HT development can be added to the score.

In some embodiments, physical dosimeters may be used to determine an actual dose of radiation delivered to the radiation-sensitive tissues. For instance, dosimeters may be attached at the skin level near bone marrow structures, such as the vertebra. At the end of each fraction the absorbed dose collected by the dosimeter is readout. Such methods may be used to complement the computational methods described herein, e.g. to confirm whether predictions concerning the received dose of radiation using the imaging data and treatment data are accurate.

In another embodiment, and specific for hadron-based radiotherapy (for example proton radiotherapy, carbon ion radiotherapy) the extent of radiation damage to the bone marrow may be determined using range detection techniques such as prompt-gamma or Compton cameras, PET-CT imaging, or PET-MRI imaging, or functional multi-parametric MRI imaging.

A computer program may be stored/distributed on a suitable medium, such as an optical storage medium or a solid-state medium supplied together with or as part of other hardware, but may also be distributed in other forms, such as via the Internet or other wired or wireless telecommunication systems.

## Claims

1. A computer-implemented method (200) for determining a risk of hematologic toxicity in a subject to be treated with radiotherapy, the method comprising:
receiving patient data (202) associated with the subject,
receiving treatment data (204), wherein the treatment data includes a prescribed dose of radiation to be administered to the subject;
receiving imaging data (206), wherein the imaging data comprises an image including one or more radiation-sensitive tissues in the subject, the radiation-sensitive tissues comprising regions of hematopoeisis and/or leukocyte maturation, activation, and/or proliferation in the subject;
processing the treatment data and the imaging data (208) to determine a received dose of radiation to be delivered to the radiation-sensitive tissues in the subject;
processing the patient data and the received dose of radiation (210) to obtain a risk of hematologic toxicity in the subject in response to the radiotherapy;
**characterised in that**:
the patient data includes blood cell counts in the subject; and
the radiation-sensitive tissue comprises bone marrow and/or lymphoid organs.

2. A method according to claim 1, wherein the hematologic toxicity comprises lymphopenia and the patient data includes lymphocyte numbers in the subject's blood.

3. A method according to claim 1 or claim 2, wherein the patient data further comprises age, gender and/or disease characteristics in the subject.

4. A method according to any preceding claim, wherein the treatment data further comprises a distribution of radiation doses to be administered to treated areas within the subject.

5. A method according to any preceding claim, wherein the imaging data is derived from computerized tomography, CT, positron emission tomography, PET, or magnetic resonance imaging, MRI.

6. A method according to any preceding claim, wherein the imaging data is indicative of proliferative and/or metabolic activity in the radiation-sensitive tissues of the subject.

7. A method according to claim 6, wherein the imaging data is derived from PET imaging using a 18F-fluorothymidine, 18F-FLT, tracer.

8. A method according to any preceding claim, wherein the radiation-sensitive tissue comprises bone marrow.

9. A method according to claim 8, wherein the radiation-sensitive tissue comprises bone marrow of the thoracic vertebrae.

10. A method according to any preceding claim, wherein the radiation-sensitive tissue includes lymphoid organs, such as thymus, lymph nodes, spleen and/or mucosa-associated lymphoid tissue.

11. A method according to any preceding claim, wherein the radiotherapy comprises fractionated radiotherapy, the treatment data includes a plurality of fraction doses, and the imaging data comprises a plurality of images, wherein each image is obtained at administration of a different radiotherapy fraction.

12. A method according to claim 11, wherein if the imaging data does not comprise an image obtained at administration of a specific radiotherapy fraction n, the method further comprises predicting imaging data corresponding to radiotherapy fraction n from the plurality of images obtained at administration of different radiotherapy fractions.

13. A method according to any preceding claim, wherein the received dose of radiation to be delivered to radiation-sensitive tissues in the image is outputted to a display.

14. A method according to any preceding claim, wherein if the risk of hematologic toxicity is determined to be above a predetermined threshold, the method further comprises receiving updated patient data including blood cell counts from the subject obtaining after initiation of the radiotherapy treatment, and obtaining an updated risk of hematologic toxicity in the subject based on the updated patient data, treatment data and imaging data.

15. A method according to any preceding claim, wherein the subject is to be treated with combined radiotherapy and immunotherapy for cancer.

16. A system (100) for determining a risk of hematologic toxicity in a subject to be treated with radiotherapy, the system comprising:
- an interface (110) for receiving:
(i) patient data (112) associated with the subject, wherein the patient data includes blood cell counts in the subject;
(ii) treatment data (114), wherein the treatment data includes a prescribed dose of radiation to be administered to the subject;
(iii) imaging data (116), wherein the imaging data comprises a graphical representation including one or more radiation-sensitive tissues in the subject, the radiation-sensitive tissues comprising regions of hematopoeisis and/or leukocyte maturation, activation, and/or proliferation in the subject; wherein the radiation-sensitive tissue comprises bone marrow and/or lymphoid organs;
- a memory (106); and
a processor (104) configured to execute instructions stored on the memory to:
(a) process the treatment data and the imaging data to determine a received dose of radiation to be delivered to the radiation-sensitive tissues in the subject; and
(b) process the patient data and the received dose of radiation to obtain a risk of hematologic toxicity in the subject in response to the radiotherapy.

17. A computer program product comprising a non-transitory computer readable medium, the computer readable medium having computer readable code embodied therein, the computer readable code being configured such that, on execution by a suitable computer or processor, the computer or processor is caused to perform the method of any of claims 1 to 15.

## Patentansprüche

1. Computer-implementiertes Verfahren (200) zum Bestimmen eines Risikos hämatologischer Toxizität bei einem mit Strahlentherapie zu behandelnden Patienten, wobei das Verfahren Folgendes umfasst:
Empfangen von Patientendaten (202), die dem Patienten zugeordnet sind, Empfangen von Behandlungsdaten (204), wobei die Behandlungsdaten eine vorgeschriebene Strahlungsdosis enthalten, die dem Patienten verabreicht werden soll;
Empfangen von Bildgebungsdaten (206), wobei die Bildgebungsdaten ein Bild umfassen, das ein oder mehrere strahlungsempfindliche Gewebe in dem Patienten enthält, wobei die strahlungsempfindlichen Gewebe Bereiche der Hämatopoese und/oder Leukozytenreifung, -aktivierung und/oder -proliferation in dem Patienten umfassen;
Verarbeitung der Behandlungsdaten und der Bildgebungsdaten (208), um eine empfangene Strahlungsdosis zu bestimmen, die an das strahlungsempfindliche Gewebe des Patienten abgegeben werden soll;
Verarbeitung der Patientendaten und der empfangenen Strahlendosis (210), um das Risiko einer hämatologischen Toxizität bei dem Patienten als Reaktion auf die Strahlentherapie zu ermitteln;
**dadurch gekennzeichnet:**
**dass** die Patientendaten die Anzahl der Blutzellen des Patienten enthalten; und
das strahlenempfindliche Gewebe umfasst Knochenmark und/oder lymphatische Organe.

2. Verfahren nach Anspruch 1, wobei die hämatologische Toxizität eine Lymphopenie umfasst und die Patientendaten die Lymphozytenzahl im Blut des Patienten umfassen.

3. Verfahren nach Anspruch 1 oder Anspruch 2, wobei die Patientendaten ferner Alter, Geschlecht und/oder Krankheitsmerkmale des Patienten umfassen.

4. Verfahren nach einem der vorhergehenden Ansprüche, wobei die Behandlungsdaten ferner eine Verteilung der Strahlungsdosen umfassen, die den behandelten Bereichen innerhalb des Patienten zu verabreichen sind.

5. Verfahren nach einem der vorhergehenden Ansprüche, wobei die Bildgebungsdaten von der Computertomographie CT, der Positronenemissionstomographie PET oder der Magnetresonanztomographie MRI abgeleitet sind.

6. Verfahren nach einem der vorhergehenden Ansprüche, wobei die Bildgebungsdaten auf eine proliferative und/oder metabolische Aktivität in den strahlungsempfindlichen Geweben des Patienten hinweisen.

7. Verfahren nach Anspruch 6, wobei die Bildgebungsdaten aus der PET-Bildgebung unter Verwendung eines 18F-Fluorothymidin, 18F-FLT, Tracers stammen.

8. Verfahren nach einem der vorhergehenden Ansprüche, wobei das strahlungsempfindliche Gewebe Knochenmark umfasst.

9. Verfahren nach Anspruch 8, wobei das strahlungsempfindliche Gewebe Knochenmark der Brustwirbel umfasst.

10. Verfahren nach einem der vorhergehenden Ansprüche, wobei das strahlungsempfindliche Gewebe lymphatische Organe, wie Thymus, Lymphknoten, Milz und/oder schleimhautassoziiertes lymphatisches Gewebe, umfasst.

11. Verfahren nach einem der vorhergehenden Ansprüche, wobei die Strahlentherapie eine fraktionierte Strahlentherapie umfasst, die Behandlungsdaten eine Vielzahl von Fraktionsdosen enthalten und die Bildgebungsdaten eine Vielzahl von Bildern umfassen, wobei jedes Bild bei der Verabreichung einer anderen Strahlentherapiefraktion erhalten wird.

12. Verfahren nach Anspruch 11, wobei, wenn die Bildgebungsdaten kein Bild umfassen, das bei der Verabreichung einer spezifischen Strahlentherapiefraktion n erhalten wurde, das Verfahren ferner die Vorhersage von Bildgebungsdaten, die der Strahlentherapiefraktion n entsprechen, aus der Vielzahl von Bildern umfasst, die bei der Verabreichung verschiedener Strahlentherapiefraktionen erhalten wurden.

13. Verfahren nach einem der vorhergehenden Ansprüche, wobei die empfangene Strahlendosis, die an strahlungsempfindliche Gewebe im Bild abgegeben werden soll, auf einem Display ausgegeben wird.

14. Verfahren nach einem der vorhergehenden Ansprüche, wobei, wenn das Risiko der hämatologischen Toxizität als über einem vorbestimmten Schwellenwert liegend bestimmt wird, das Verfahren ferner das Empfangen aktualisierter Patientendaten, einschließlich Blutzellzahlen, die von dem Patienten nach
Beginn der Strahlentherapiebehandlung erhalten wurden, und dadurch das Erhalten eines aktualisierten Risikos für die hämatologische Toxizität bei dem Patienten auf der Grundlage der aktualisierten Patientendaten, Behandlungsdaten und Bildgebungsdaten umfasst.

15. Verfahren nach einem der vorhergehenden Ansprüche, wobei der Patient mit einer kombinierten Strahlen- und Immuntherapie gegen Krebs behandelt werden soll.

16. System (100) zur Bestimmung des Risikos einer hämatologischen Toxizität bei einem mit Strahlentherapie zu behandelnden Patienten, wobei das System Folgendes umfasst:
- eine Schnittstelle (110) für den Empfang:
(i) Patientendaten (112), die dem Patienten zugeordnet sind, wobei die Patientendaten die Blutzellzahlen des Patienten umfassen;
(ii) Behandlungsdaten (114), wobei die Behandlungsdaten eine vorgeschriebene Strahlungsdosis enthalten, die dem Patienten verabreicht werden soll;
(iii) Bildgebungsdaten (116), wobei die Bildgebungsdaten eine grafische Darstellung umfassen, die ein oder mehrere strahlungsempfindliche Gewebe in dem Patienten einschließt, wobei die strahlungsempfindlichen Gewebe Bereiche der Hämatopoese und/oder Leukozytenreifung, - aktivierung und/oder -proliferation in dem Patienten umfassen; wobei das strahlungsempfindliche Gewebe Knochenmark und/oder lymphoide Organe umfasst;
- einen Speicher (106); und
einen Prozessor (104), der so konfiguriert ist, dass er im Speicher gespeicherte Anweisungen ausführt, um:
(a) die Behandlungsdaten und die Bildgebungsdaten zu verarbeiten, um eine empfangene Strahlungsdosis zu bestimmen, die den strahlungsempfindlichen Geweben in der Person zugeführt werden soll; und
(b) die Patientendaten und die erhaltene Strahlendosis zu verarbeiten, um das Risiko einer hämatologischen Toxizität bei dem Patienten als Reaktion auf die Strahlentherapie zu ermitteln.

17. Computerprogrammprodukt, das ein nichttransitorisches computerlesbares Medium umfasst, wobei das computerlesbare Medium einen darin verkörperten computerlesbaren Code aufweist und der computerlesbare Code so konfiguriert ist, dass bei Ausführung durch einen geeigneten Computer oder Prozessor der Computer oder Prozessor veranlasst wird, das Verfahren nach einem der Ansprüche 1 bis 15 auszuführen.

## Revendications

1. Procédé mis en œuvre par ordinateur (200) pour déterminer un risque de toxicité hématologique chez un sujet devant être traité par radiothérapie, le procédé comprenant:
recevoir des données de patient (202) associées au sujet, recevoir des données de traitement (204), dans lequel les données de traitement comprennent une dose prescrite de rayonnement à administrer au sujet;
recevoir des données d'imagerie (206), dans lequel les données d'imagerie comprennent une image incluant un ou plusieurs tissus sensibles au rayonnement dans le sujet, les tissus sensibles au rayonnement comprenant des régions d'hématopoïèse et/ou de maturation, d'activation, et/ou de prolifération de leucocytes dans le sujet;
traiter les données de traitement et les données d'imagerie (208) pour déterminer une dose reçue de rayonnement à délivrer aux tissus sensibles au rayonnement dans le sujet;
traiter les données du patient et la dose reçue de rayonnement (210) pour obtenir un risque de toxicité hématologique chez le sujet en réponse à la radiothérapie;
**caractérisé en ce que**
les données du patient comprennent des numérations de cellules sanguines chez le sujet; et
le tissu sensible au rayonnement comprend la moelle osseuse et/ou les organes lymphoïdes.

2. Procédé selon la revendication 1, dans lequel la toxicité hématologique comprend la lymphopénie et les données du patient comprennent le nombre de lymphocytes dans le sang du sujet.

3. Procédé selon la revendication 1 ou la revendication 2, dans lequel les données du patient comprennent en outre l'âge, le sexe et/ou les caractéristiques de la maladie chez le sujet.

4. Procédé selon l'une quelconque des revendications précédentes, dans lequel les données de traitement comprennent en outre une distribution des doses de rayonnement à administrer aux zones traitées dans le sujet.

5. Procédé selon l'une quelconque des revendications précédentes, dans lequel les données d'imagerie sont dérivées de la tomographie informatisée, CT, de la tomographie par émission de positons, TEP, ou de l'imagerie par résonance magnétique, IRM.

6. Procédé selon l'une quelconque des revendications précédentes, dans lequel les données d'imagerie sont indicatives d'une activité proliférative et/ou métabolique dans les tissus sensibles aux rayonnements du sujet.

7. Procédé selon la revendication 6, dans lequel les données d'imagerie sont dérivées d'une imagerie TEP utilisant un traceur 18F-fluorothymidine, 18F-FLT.

8. Procédé selon l'une quelconque des revendications précédentes, dans lequel le tissu sensible aux rayonnements comprend de la moelle osseuse.

9. Procédé selon la revendication 8, dans lequel le tissu sensible au rayonnement comprend la moelle osseuse des vertèbres thoraciques.

10. Procédé selon l'une quelconque des revendications précédentes, dans lequel le tissu sensible aux rayonnements comprend des organes lymphoïdes, tels que le thymus, les ganglions lymphatiques, la rate et/ou le tissu lymphoïde associé aux muqueuses.

11. Procédé selon l'une quelconque des revendications précédentes, dans lequel la radiothérapie comprend une radiothérapie fractionnée, les données de traitement comprennent une pluralité de doses fractionnées, et les données d'imagerie comprennent une pluralité d'images, dans lequel chaque image est obtenue lors de l'administration d'une fraction de radiothérapie différente.

12. Procédé selon la revendication 11, dans lequel si les données d'imagerie ne comprennent pas une image obtenue lors de l'administration d'une fraction de radiothérapie spécifique n, le procédé comprend en outre la prédiction de données d'imagerie correspondant à la fraction de radiothérapie n à partir de la pluralité d'images obtenues lors de l'administration de différentes fractions de radiothérapie.

13. Procédé selon l'une quelconque des revendications précédentes, dans lequel la dose reçue de rayonnement à délivrer aux tissus sensibles au rayonnement dans l'image est sortie sur un écran.

14. Procédé selon l'une quelconque des revendications précédentes, dans lequel si le risque de toxicité hématologique est déterminé comme étant supérieur à un seuil prédéterminé, le procédé comprend en outre la réception de données de patient mises à jour comprenant des numérations de cellules sanguines du sujet obtenues après le début du traitement de radiothérapie, et l'obtention d'un risque mis à jour de toxicité hématologique chez le sujet sur la base des données de patient mises à jour, des données de traitement et des données de l'imagerie.

15. Procédé selon l'une quelconque des revendications précédentes, dans lequel le sujet doit être traité par radiothérapie et immunothérapie combinées pour le cancer.

16. Système (100) pour déterminer un risque de toxicité hématologique chez un sujet devant être traité par radiothérapie, le système comprenant:
- une interface (110) pour recevoir:
(i) des données de patient (112) associées au sujet, dans lesquelles les données de patient comprennent des numérations de cellules sanguines chez le sujet;
(ii) des données de traitement (114), dans lesquelles les données de traitement comprennent une dose prescrite de rayonnement à administrer au sujet;
(iii) des données d'imagerie (116), dans lesquelles les données d'imagerie comprennent une représentation graphique incluant un ou plusieurs tissus sensibles au rayonnement chez le sujet, les tissus sensibles au rayonnement comprenant des régions d'hématopoïèse et/ou de maturation, d'activation, et/ou de prolifération de leucocytes chez le sujet; dans lesquelles le tissu sensible au rayonnement comprend la moelle osseuse et/ou les organes lymphoïdes;
- une mémoire (106); et
un processeur (104) configuré pour exécuter des instructions stockées sur la mémoire pour:
(a) traiter les données de traitement et les données d'imagerie pour déterminer une dose reçue de rayonnement à délivrer aux tissus sensibles au rayonnement dans le sujet; et
(b) traiter les données du patient et la dose reçue de rayonnement pour obtenir un risque de toxicité hématologique chez le sujet en réponse à la radiothérapie.

17. Produit de programme informatique comprenant un support lisible par ordinateur non transitoire, le support lisible par ordinateur comportant un code lisible par ordinateur incorporé dans celui-ci, le code lisible par ordinateur étant configuré de telle sorte que, lors de l'exécution par un ordinateur ou un processeur approprié, l'ordinateur ou le processeur est amené à exécuter le procédé de l'une quelconque des revendications 1 à 15.
